# EUROPÄISCHE PATENTSCHRIFT

(11) **EP 3 103 391 B1**
(45) Veröffentlichungstag und Bekanntmachung des Hinweises auf die Patenterteilung: **10.10.2018**
(21) Anmeldenummer: 16166557.5
(22) Anmeldetag: 22.04.2016
(51) Int. Cl.: A61B 5/1455, A61N 1/365, A61B 5/042, A61B 5/0245, A61B 5/053, A61B 5/11, A61B 5/1459, A61N 1/39, A61B 5/01

(54) **IMPLANTIERBARE VORRICHTUNG MIT EINEM SAUERSTOFFSENSOR**
IMPLANTABLE DEVICE WITH AN OXYGEN SENSOR
DISPOSITIF IMPLANTABLE COMPRENANT UN CAPTEUR D'OXYGÈNE

(30) Priorität: 21.05.2015 US 201562164614 P
(43) Veröffentlichungstag der Anmeldung: 14.12.2016
(73) Patentinhaber: BIOTRONIK SE & Co. KG, 12359 Berlin (DE)
(72) Erfinder: Dörr, Thomas, 12437 Berlin (DE)
(74) Vertreter: Galander, Marcus

(56) Entgegenhaltungen:
- EP-A2- 0 793 975
- WO-A1-96/16696
- WO-A1-2010/088539
- WO-A2-2004/066814
- FR-A1- 2 903 912
- US-A- 5 342 406
- US-A1- 2006 247 702
- US-A1- 2010 022 856
- US-A1- 2010 030 043
- US-A1- 2010 280 348
- US-A1- 2011 009 754

## Beschreibung

Die Erfindung betrifft eine implantierbare Vorrichtung mit einem Sauerstoffsensor.

Sauerstoffsensoren zur Implantation in den menschlichen oder tierischen Körper sind bekannt, sowohl für die Messung des Sauerstoffgehalts im Blut, d.h. in mit Blut gefüllten Hohlräumen wie Herzkammern oder Blutgefäßen, als auch im Gewebe, soweit dieses durchblutet ist. Bei optischen Sensoren sind drei Anordnungen bekannt. Beim Durchlichtverfahren ist das Blut oder Gewebe zwischen Quelle und Detektor angeordnet. Beim Streulichtverfahren leuchtet die Quelle in das Gewebe oder Blut, und der Detektor nutzt das rückgestrahlte Spektrum. Beim Reflexionsverfahren reflektiert ein Spiegel das von der Quelle ausgesandte Licht auf den Detektor, und das zu untersuchende Blut oder Gewebe befindet sich in den beiden Lichtwegen Quelle-Spiegel und Spiegel-Detektor. Beispiele hierzu sind z.B. aus US 20100022856 A1, US 20100030043 A1, US 20130289372 A1, US 20070239215 A1, US 20070239052 A1 und US 20110190610 A1 bekannt.

Der Erfindung liegt die Aufgabe zugrunde, eine einfache und zuverlässige dauerimplantierbare Vorrichtung zu schaffen, mit welcher ein Sauerstoffgehalt in Blut oder Gewebe bestimmt werden kann.

Eine weitere Aufgabe der Erfindung besteht darin, eine Vorrichtung zur Bestimmung der Sauerstoffextraktionsrate des Herzens anzugeben, das eine schnell greifende therapeutische Maßnahme erlaubt.

Die Aufgabe wird erfindungsgemäß durch die Merkmale des unabhängigen Anspruchs gelöst. Günstige Ausgestaltungen und Vorteile der Erfindung ergeben sich aus den abhängigen Ansprüchen.

Es wird nach einem Aspekt der Erfindung eine Vorrichtung entsprechend Anspruch 1 vorgeschlagen.

Zur Detektion von Sauerstoff im Gewebe oder im Blut kann jede geeignete Äquivalenzgröße herangezogen werden, die abhängig von einem aktuellen Sauerstoffgehalt in Körpergewebe oder Blut ist. Beispielsweise kann dies eine Transmission und/oder Absorption für elektromagnetische Signale in einem gegebenen Frequenzbereich sein, insbesondere im infraroten oder optischen Frequenzbereich. Im Folgenden bezieht sich der Zusatz "optisch" sowohl auf den sichtbaren Bereich als auch auf den infraroten Bereich des elektromagnetischen Frequenzspektrums. Die Sensoreinheit kann insbesondere eine optische Sensoreinheit sein, bevorzugt ein Oxymeter, die im Frequenzbereich zwischen 600 nm und 1000 nm, insbesondere zwischen 660 nm und 940 nm, arbeitet. Vorteilhaft ist ein solches Oxymeter aus einer oder mehreren Leucht- oder Laserdioden als Sender und einem Phototransistor oder Photodiode oder Photowiderstand als Empfänger gebildet. Eine rote und eine infrarote Lichtquelle erlauben es, die lokale Sauerstoffsättigung in der Messstrecke zu bestimmen.

Die Vorrichtung erlaubt insbesondere eine Verbesserung der Therapie herzinsuffizienter Patienten. Bekannte Vorrichtungen und Verfahren betrachten primär das maximale Herzzeitvolumen ("Cardiac Output") als Optimierungsziel. Vorteilhaft ist es jedoch, dabei auch den myokardialen Sauerstoffverbrauch in Form einer Sauerstoffextraktionsrate bei definierten Belastungssituationen zu berücksichtigen und möglichst zu verbessern. Damit kann eine Progression der Herzinsuffizienz bis zur Dekompensation vermieden oder zumindest verzögert werden. Zum Beispiel ist die Blutversorgung des linken Ventrikels im Bereich der submyokardialen Koronarien durch Perfusion während der Systole stark vermindert, da die Koronargefäße durch die Kontraktion der Arbeitsmuskulatur stark komprimiert und damit vorübergehend verschlossen werden. Mit steigender Herzfrequenz steigt auch der zeitliche Anteil der linksventrikulären Systole bezogen auf den gesamten Herzzyklus. Mit der erfindungsgemäßen Vorrichtung gelingt eine dauerhafte Überwachung der myokardialen Sauerstoffbilanz, was es ermöglicht, eine medikamentöse und/oder elektrische Therapie an den jeweiligen kardialen Status anzupassen. So kann zum Beispiel die Stimulationsfrequenz eines Herzschrittmachers reduziert werden, oder aber eine Herzunterstützungspumpe in ihrer Leistung gesteigert werden oder eine CCM-Stimulation (Cardiac Contractility Modulation) aktiviert werden oder auch die Dosierung eines Betablockers oder anderer Medikamente angepasst werden oder in erweiterten Notfallsystemen automatisch Dobutamin verabreicht werden.

Die myokardiale Sauerstoffextraktionsrate beschreibt dabei die prozentuale Differenz des Sauerstoffgehaltes zwischen Koronararterien und Koronarvenen. Bei sinkendem Sauerstoffangebot steigt die Sauerstoffextraktionsrate zunächst an, bis deren gewebetypisches Maximum erreicht wird. Ab diesem Punkt ist von einer beginnenden Gewebshypoxie mit darauffolgender Dekompensation auszugehen. Die Vorrichtung erlaubt es, diesen Punkt zu erkennen und rechtzeitig therapeutisch einzugreifen.

Die Erfindung ermöglicht es, mit einem implantierbaren Sensor die myokardiale Sauerstoffextraktionsrate im Verlauf insbesondere bei herzinsuffizienten Patienten kontinuierlich zu überwachen und den Therapiebedarf entsprechend anzupassen. Es kann dauerhaft eine Gewebesauerstoffsättigung bestimmt werden. Weiterhin können zu diagnostischen Zwecken oder Zwecken der Therapiesteuerung elektronische Implantate eingesetzt werden, die mit der Vorrichtung funktionell und/oder baulich gekoppelt sind.

Es ist eine dauerimplantierbare Überwachung der myokardialen Sauerstoffbilanz (Sauerstoffextraktionsrate) zur Verlaufskontrolle und Therapiesteuerung bei herzinsuffizienten Patienten möglich.

Der Reflektor ist an oder in einer Fixiervorrichtung zur dauerhaften Fixierung der Vorrichtung an oder in einem Körpergewebe angeordnet. Dabei kann auf erprobte Fixiermechanismen an einer Gewebestruktur zurückgegriffen werden. Die Vorrichtung kann dauerhaft am oder im Körpergewebe fixiert werden. Gleichzeitig können die für die Messung notwendigen Baugruppen geeignet positioniert und fixiert werden, ohne dass hierfür zusätzliche Implantationsschritte notwendig sind. Vorteilhaft kann die Fixiervorrichtung eine Helix zur dauerhaften Fixierung der Elektrodenleitung im Körpergewebe sein. Ein Teil oder die gesamte Fixiereinrichtung kann verspiegelt oder poliert sein, so dass diese zusätzlich einen Reflektor für die Signale darstellen, welche der Sender zum Empfänger sendet, um eine sauerstoffsensitive Messung, etwa eine Sauerstoffsättigungsmessung, durchzuführen. Nach einer günstigen Ausgestaltung kann die Sensoreinheit mit einem oder mehreren therapeutischen Implantaten gekoppelt oder koppelbar sein. Die Messwerte der Sensoreinheit können mittels einer Auswerteeinheit ausgewertet und gegebenenfalls bewertet werden, so dass kritische Bereiche z.B. einer Sauerstoffextraktionsrate und/oder einer Sauerstoffsättigung erkannt werden können.

Nach einer günstigen Ausgestaltung kann die Vorrichtung die Sensoreinheit sowie eine mit der Sensoreinheit gekoppelte Therapieeinheit umfassen. Alternativ oder zusätzlich kann die Sensoreinheit Bestandteil einer Therapieeinheit sein, welche wenigstens eine Wahrnehmungselektrode und/oder eine therapeutische Stimulationselektrode und/oder eine Defibrillationselektrode umfasst. Wird ein kritischer Messwert oder Bereich erkannt, kann über die Therapieeinheit eine therapeutische Maßnahme ergriffen werden.

Nach einer günstigen Ausgestaltung kann die Sensoreinheit mit einer Einrichtung zur Erfassung eines pH-Werts gekoppelt sein. Die Einrichtung kann auch in die Sensoreinheit integriert sein. Dadurch, dass zusätzlich der pH-Wert bestimmt wird, können z.B. Sauerstoffsättigungswerte im Körpergewebe oder im Blut entsprechend korrigiert werden. Die Messgenauigkeit kann somit erhöht werden.

Nach einer günstigen Ausgestaltung kann die Sensoreinheit mit einem Temperatursensor oder mit einem Temperatursensor und einem Heizelement gekoppelt sein. Mit einem Temperatursensor können temperaturabhängige Effekte im Bereich der Messstrecke, welche das Messergebnis verfälschen können, kompensiert werden. Der Temperatursensor kann ein Thermistor, ein Thermoelement, eine P-N Diode, ein bipolarer Transistor und der gleichen sein.

Ist zusätzlich ein Heizelement vorgesehen, kann eine definierte Temperatur im Bereich der Messstrecke eingestellt werden, um Temperatureffekte bei der Messung zu eliminieren. Es kann gezielt ein Bereich des Körpergewebes erwärmt werden. Dazu können auch mehrere Einzelelemente über einen weiten Bereich verteilt werden, um eine möglichst konstante Temperaturverteilung zu erreichen. Das Heizelement kann z.B. ein Heizwiderstand sein, der gesteuert oder geregelt werden kann, um eine unerwünschte Überhitzung des Körpergewebes oder eine Beeinflussung der Elektronik der Vorrichtung oder von anderen Implantaten zu vermeiden. Das Heizelement kann z.B. an einem Gehäuse der Vorrichtung oder einem anderen Implantat angeordnet sein.

Weiterhin ist es möglich, durch eine Erwärmung des Körpergewebes eine gezielte Gefäßerweiterung zu bewirken, um z.B. einen arteriellen Beitrag zur lokalen Bestimmung des Sauerstoffgehalts gezielt zu erhöhen. Generell können arterielles, venöses und Kapillarblutvolumen im Körpergewebe zur Messung beitragen. Bei manchen Messungen sind arterielle oder venöse Beiträge unerwünscht, etwa bei der Messung des Sauerstoffgehalts in der Skelettmuskulatur.

Nach einer günstigen Ausgestaltung kann die Sensoreinheit mit einer Einrichtung zur Erfassung eines Kontraktionszustands von Körpergewebe im Bereich der Messstrecke gekoppelt sein. Insbesondere kann die Einrichtung zur Erfassung des Kontraktionszustands einen mechanischen Kontraktionszustand erfassen und z.B. einen oder mehrere Dehnungsmessstreifen, einen oder mehrere Drucksensoren, einen oder mehrere Beschleunigungssensoren aufweisen. Ebenso kann eine Temperaturmessung zur Bestimmung des Kontraktionszustands herangezogen werden. Beim Herzen können auch Herztöne zur Bestimmung des Kontraktionszustands herangezogen werden. Ebenso ist eine Kombination von zwei oder mehreren der Methoden möglich, um die Messgenauigkeit zu erhöhen. Vorteilhaft ist die Erfassung des mechanischen Kontraktionszustands des Herzens, um zwischen Systole und Diastole zu unterscheiden. Ebenso kann der mechanische Kontraktionszustand mittels einer unipolaren, kontinuierlichen Impedanzmessung erfasst werden, um aus diesen Signalen Beginn und Ende der Kontraktionszustände zu bestimmen. Dies kann in einer Klassifiziereinheit erfolgen. Ist die Sensoreinheit mit einer solchen Einrichtung zur Erfassung des Kontraktionszustands gekoppelt oder weist sie eine solche Einrichtung auf, kann das auf einen Sauerstoffgehalt reagierende Signal einem bestimmten Kontraktionszustand zugeordnet werden. So können die Phasen der mechanischen Pumpfunktion des Herzens der Sauerstoffsättigung zeitlich zugeordnet werden, was in einer Synchronisationseinheit erfolgen kann. In einer Auswerteeinheit kann daraus anschließend der Sauerstoffverlauf hinsichtlich eines Hypoxiekriteriums (z.B. übermäßiges Absinken am Ende der Systole bezogen auf den mittleren Sauerstoffwert über den gesamten Herzzyklus) ausgewertet und signalisiert werden.

Nach einer günstigen Ausgestaltung kann die Sensoreinheit zusätzlich mit einer Elektrodenleitung zur Wahrnehmung der elektrischen Aktivität des Herzens verbunden sein, um so die kardiale Zykluslänge zu bestimmen. Hierdurch kann das Signal, welches repräsentativ für einen Sauerstoffgehalt ist, jeweils für einen Herzzyklus und/oder bestimmte Phasen eines Herzzyklus ermittelt werden. Vorteilhaft ist die Erfassung der elektrischen Aktivität des Herzens weiterhin, um zwischen Systole und Diastole zu unterscheiden.

Nach einer günstigen Weiterentwicklung der Erfindung werden aus dem Signal, welches repräsentativ für einen Sauerstoffgehalt ist, eine maximale myokardiale Sauerstoffsättigung, eine minimale myokardiale Sauerstoffsättigung und eine Differenz zwischen minimaler und maximaler myokardialer Sauerstoffsättigung bestimmt und daraus eine Sauerstoffextraktionsrate des Herzens abgeleitet.

Nach einer vorteilhaften Ausgestaltung der Erfindung wird aus dem Signal, welches repräsentativ für einen Sauerstoffgehalt ist, die maximale, minimale und die Differenz der myokardiale Sauerstoffsättigung über einen Herzzyklus bestimmt und daraus ein Verlauf einer Sauerstoffextraktionsrate in Abhängigkeit der Herzfrequenz bestimmt.

Nach einer günstigen Ausgestaltung kann aus einem Verlauf der gemessenen Sauerstoffextraktionsrate über der Herzfrequenz ein kritischer Punkt der Sauerstoffextraktionsrate bestimmt werden, bei dem die Sauerstoffextraktionsrate bei steigender Herzfrequenz absinkt; und/oder wenn die Sauerstoffextraktionsrate bei steigender Herzfrequenz in einen Sättigungsbereich übergeht; und/oder wenn die Sauerstoffextraktionsrate bei steigender Herzfrequenz in einen Sättigungsbereich übergeht und gleichzeitig der maximale Sauerstoffsättigungswert absinkt.

Nach einem weiteren Aspekt der Erfindung wird ein kritischer Punkt der Sauerstoffextraktionsrate bestimmt, ab dem eine systolische Sauerstoffsättigung unter einen kritischen Referenzwert absinkt oder die Absinkgeschwindigkeit einen kritischen Wert während der Systole übersteigt.

Nach einem weiteren Aspekt der Erfindung wird ein Herzunterstützungssystem entsprechend Anspruchs 13 vorgeschlagen.

Nach einer günstigen Ausgestaltung des Herzunterstützungssystems kann die Sensoreinheit mit einer Einrichtung zur Erfassung eines Kontraktionszustands des Herzens gekoppelt sein.

Nach einer günstigen Ausgestaltung des Herzunterstützungssystems kann ein oder mehrere Stimulations- und/oder Therapieparameter derart optimiert werden, dass eine Sauerstoffsättigung bezogen auf einen definierten Belastungszustand des Herzens eingestellt wird.

Die Vorrichtung mit der Sensoreinheit kann hierzu zweckmäßigerweise mit einer geeigneten Auswerteeinheit verbunden sein. Diese kann den kritischen Punkt der gemessenen Sauerstoffextraktionsrate bestimmen, wenn diese bei steigender Herzfrequenz absinkt. Sie kann ferner den kritischen Punkt der gemessenen Sauerstoffextraktionsrate bestimmen, wenn diese bei steigender Herzfrequenz in einen Sättigungsbereich übergeht. Ferner kann die Auswerteeinheit den kritischen Punkt der gemessenen Sauerstoffextraktionsrate bestimmen, wenn diese bei steigender Herzfrequenz in einen Sättigungsbereich übergeht und gleichzeitig der maximale Sauerstoffsättigungswert absinkt. Die Auswerteeinheit kann insbesondere die maximale, minimale und die Differenz der myokardialen Sauerstoffsättigung über einen Herzzyklus bestimmen und den Verlauf dieser so bestimmten Sauerstoffextraktionsrate herzfrequenzbezogen aufzeichnen. Basierend auf dem dergestalt bestimmten Trend der Differenzwerte können bei Bedarf therapeutische Maßnahmen eingeleitet werden.

Die Auswerteeinheit kann zusätzlich mit einer Elektrodenleitung zur Wahrnehmung der elektrischen Aktivität des Herzens verbunden sein, um so die kardiale Zykluslänge zu bestimmen.

Die Erfindung ermöglicht es, in einer dauerimplantierbaren Vorrichtung die myokardiale Sauerstoffversorgung spezifisch zu überwachen und den Zeitpunkt einer einsetzenden myokardialen Hypoxie, insbesondere bei herzinsuffizienten Patienten frühzeitig zu erkennen und automatisch oder manuell die Therapie anzupassen.

Die Erfindung ist nachfolgend beispielhaft, anhand von Zeichnungen näher erläutert. Es zeigen in schematischer Darstellung:
- Fig. 1: einen Schnitt durch ein Herz mit einer dauerimplantierten Vorrichtung, welche in einem Herzunterstützungssystem integriert ist;
- Fig. 2: einen Schnitt durch einen Elektrodenkopf mit einer Sensoreinheit mit Sender und Empfänger so wie Reflektor an einer Fixiereinrichtung des Elektrodenkopfs;
- Fig. 3: ein Detail des Elektrodenkopfs aus Figur 2;
- Fig. 4: einen Verlauf einer myokardialen Sauerstoffextraktionsrate in Abhängigkeit eines sinkenden Sauerstoffangebots;
- Fig. 5: einen Verlauf einer Sauerstoffextraktionsratenmessgröße in Abhängigkeit einer Herzfrequenz;
- Fig. 6: ein Blockschaltbild einer Vorrichtung mit einer Sensoreinheit;
- Fig. 7: einen Verlauf einer myokardialen Sauerstoffsättigung in Abhängigkeit eines Kontraktionszustands in einem nichthypoxischen Bereich;
- Fig. 8: einen Verlauf einer myokardialen Sauerstoffsättigung in Abhängigkeit eines Kontraktionszustands in einem hypoxischen Bereich; und
- Fig. 9: ein Blockschaltbild einer Vorrichtung mit einer Sensoreinheit.

- Fig. 10: ein Blockschaltbild einer Vorrichtung mit einer Sensoreinheit.

In den Figuren sind funktionell gleiche oder gleich wirkende Elemente jeweils mit denselben Bezugszeichen beziffert. Die Figuren sind schematische Darstellungen. Sie bilden nicht spezifische Parameter ab. Weiterhin geben die Figuren lediglich typischen Ausgestaltungen wieder und sollen die Erfindung nicht auf die dargestellten Ausgestaltungen beschränken.

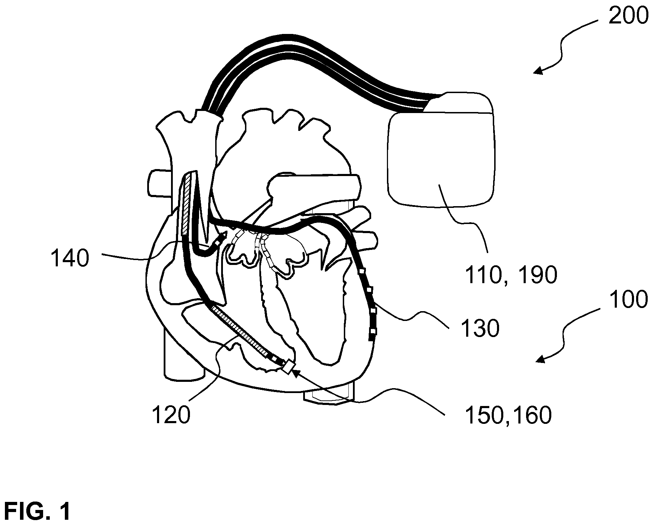

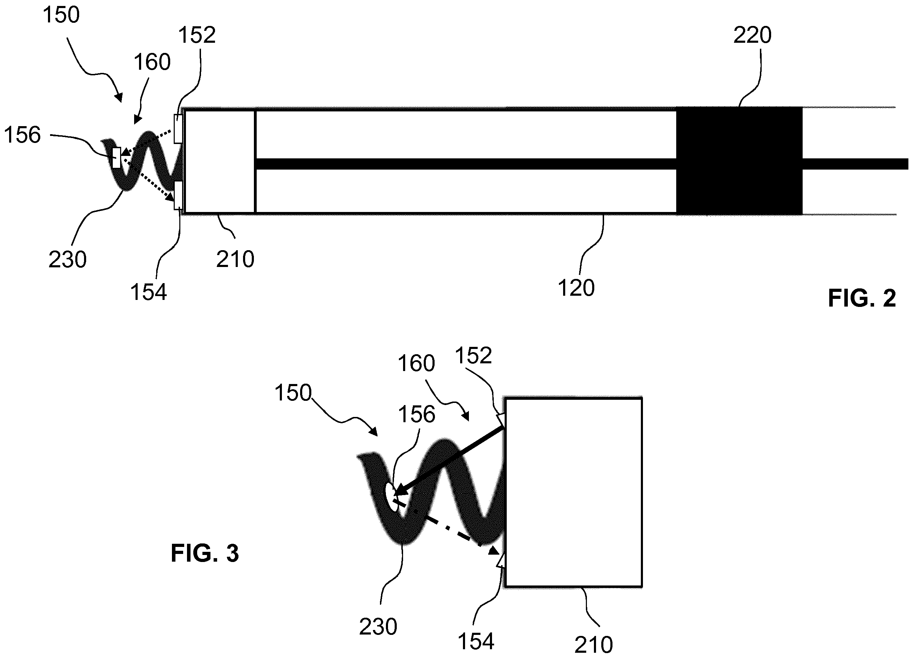

Figur 1 zeigt einen Schnitt durch ein Herz mit einer in einen menschlichen oder tierischen Körper dauerimplantierten Vorrichtung 100, welche in einem Herzunterstützungssystem 200 integriert ist. Die Vorrichtung 100 umfasst wenigstens eine Sensoreinheit 150, die zum Detektieren eines Signals vorgesehen ist, welches repräsentativ für einen Sauerstoffgehalt entlang einer Messstrecke 160 der Sensoreinheit 150 ist, welche beispielhaft in den Figuren 2 und 3 näher dargestellt ist.

Das Herzunterstützungssystem 200 umfasst einen Pulsgenerator 110 mit einer Steuereinheit 190, an den eine rechtsventrikuläre Wahrnehmungs-, Stimulations- und Schockelektrodenleitung 120, eine linksventrikuläre quadrupolare Wahrnehmungs- und Stimulationselektrodenleitung 130 und eine atriale Wahrnehmungs- und Stimulationselektrodenleitung 140 angeschlossen sind. Die elektrische Versorgung des Herzunterstützungssystems 200 kann in den Pulsgenerator 110 integriert sein oder von außen erfolgen. Die Sensoreinheit 150 ist beispielsweise im Elektrodenkopf am distalen Ende der rechtsventrikulären Wahrnehmungs-, Stimulations- und Schockelektrodenleitung 120 angeordnet und in Form eines optischen myokardialen Oxymetriesensors mit einem LED-Phototransistor-System mit Sender und Empfänger (Sender 152, Empfänger 154 in Figuren 2 und 3) ausgebildet.

Die Sensoreinheit 150 ist dabei am distalen Ende der rechtsventrikulären Elektrodenleitung 120 derart angeordnet, dass dieser nach Implantation der Elektrode ausschließlich die myokardiale Sauerstoffsättigung bestimmt. Dies wird erreicht, indem das LED-Phototransistor-System so angeordnet ist, dass dieses nach Implantation nur in das Myokard hineinstrahlen und die entsprechende Reflektion erfassen kann. Üblicherweise sind optische Oxymeter aus zwei LEDs mit einer Wellenlänge von 660 nm und 940 nm als Sender 152 und einem Phototransistor als Empfänger 154 aufgebaut. Durch moderne Integrationsmethoden können diese Strukturen im Elektrodenkopf untergebracht werden.

Das Herzunterstützungssystem 200 ist in der Figur 1 als CRT-D-System aufgebaut. CRT bedeutet Cardiac Resynchronization Therapy (kardiale Resynchronisations-Therapie), wobei ein CRT-D-System einen Defibrillator aufweist. Das Herzunterstützungssystem 200 kann auch als CRT-P-System mit einem Schrittmacher aufgebaut sein oder als VAD-System (ventricular assist device), das als ventrikuläres Unterstützungssystem einen Teil oder die gesamte Pumpfunktion des Herzens übernimmt, oder als CCM-Stimulator (cardiac contractility modulator), der als kardialer Kontraktilitätsmodulator den Herzmuskel mit nicht-erregenden elektrischen Signalen stimuliert. Das Herzunterstützungssystem 200 kann auch als aortale Ballonpumpe ausgebildet sein. Ebenso sind Kombinationen von zwei oder mehr der verschiedenen Systeme als Herzunterstützungssystem 200 denkbar.

Figur 2 zeigt einen Schnitt durch den distal angeordneten Elektrodenkopf der rechtsventrikulären Elektrodenleitung 120 aus Figur 1 mit der Sensoreinheit 150, die als optische Einheit ausgebildet ist, und Figur 3 zeigt ein Detail des Elektrodenkopfs aus Figur 2. Der Elektrodenkopf kann Elektrodenpole, wie zum Beispiel eine Tip-Elektrode 210 und/oder eine oder mehrere Ringelektroden 220 umfassen. Die hier dargestellte Elektrode 120 besitzt zusätzlich eine Helix 230 zur dauerhaften Fixierung der Elektrodenleitung 120 im Körpergewebe (hier z.B. Myokard). Alternativ oder zusätzlich zur Tip-Elektrode 210 kann auch die Helix 230 als Elektrodenpol verwendet werden. Zusätzlich ist das distale Ende der Elektrode 120 mit zwei LED als Sender 152 und einem Phototransistor als Empfänger 154 bestückt, die zusammen ein optisches Oxymeter ergeben, welches das Gewebe entlang der Messstrecke 160 erfasst. Um nun mit einer minimalen Leistung dieses System sehr zuverlässig betreiben zu können, ist ein Teil oder die gesamte Fixiereinrichtung verspiegelt oder poliert, so dass diese zusätzlich einen Reflektor 156 für die roten und infraroten Strahlen zur Sauerstoffsättigungsmessung entlang der Messstrecke 160 darstellt.

Statt einer Helix 230 zur dauerhaften Fixierung im oder am Körpergewebe sind auch andere Verankerungsmittel möglich, etwa ein Widerhaken oder dergleichen.

Mit diesem Reflektor 156 wird auch erreicht, dass bei dieser Einbauweise überwiegend nur der Myokard-Sauerstoff bestimmt wird.

In der Figur 3 ist der Elektrodenkopf aus Abbildung 2 vergrößert dargestellt. Das Gehäuse des Elektrodenkopfes enthält die Fixierhelix 230 als feste oder ausschraubbare Helix. Für die optische Oxymetrie sind eine oder mehrere LEDs als Sender 152 derart auf dem Elektrodenkopf positioniert, dass diese ihren Fokus auf eine polierte Fläche der Fixierhelix 230 ausrichten. Diese polierte Fläche bildet den Reflektor 156 auf der Helix 230 und ist derart angeordnet, dass diese im ausgeschraubten Zustand das Licht an den als Empfänger 154 dienenden Phototransistor reflektiert und so eine robuste Blutsauerstoffmessung möglich wird.

Dabei kann gleichzeitig eine Einheit zur Bestimmung des pH-Werts vorgesehen sein und die gemessene den Sauerstoffgehalt repräsentierende Größe, z.B. ein die Sauerstoffsättigung repräsentierendes optisches Signal, entsprechend korrigiert werden. Diese pH-Wert Kompensation ist insbesondere für Anwendungen mit einer hohen Signalauflösung notwendig. Der sog. Bohr-Effekt beschreibt eine pH-abhängige Sauerstoffbindungskapazität des Blutes. Bei sinkendem pH-Wert sinkt auch die Sauerstoffbindung im Blut.

Eine solche Sensoreinheit 150 kann für verschiedene Anwendungen genutzt werden.

So kann die Sensoreinheit 150 für ein Oxymeter-gesteuertes CRT-Timing eingesetzt werden, bei dem ein implantierbarer kardialer Stimulator, vorzugsweise ein CRT-Stimulator, dessen Stimulationsparameter basierend auf einer myokardialen Sauerstoffsättigungsmessung mit einer Sensoreinheit 150 optimiert werden. Die Sauerstoffsättigung muss dabei mindestens über einen gesamten kardialen Zyklus bestimmt werden. Die Optimierung stellt dabei eine minimale Differenz zwischen maximaler und minimaler myokardialer Sauerstoffsättigung SpO₂ bei definiertem Herzzeitvolumen (Cardiac Output) ein.

Ferner kann die Sensoreinheit 150 für eine Oxymeter-gesteuerte Defibrillation eingesetzt werden, bei der basierend auf der gemessenen myokardialen Sauerstoffsättigung oder deren Verlauf seit Beginn einer primär frequenzdetektierten Tachykardie der Zeitpunkt der Defibrillationsschockabgabe festgelegt wird, indem die Schocktherapie immer dann oder nur dann abgegeben wird, wenn die myokardiale Sauerstoffsättigung unterhalb eines bestimmten Grenzwertes bzw. prozentual gesunken ist.

Weiterhin kann die Sensoreinheit 150 für Oxymeter-gesteuerte Frequenzanpassung und/oder zur Vermeidung unnötiger Stimulation bei kabellosen Schrittmachern (iLP = Injectable Leadless Pacer oder LLP = Leadless Pacer) eingesetzt werden. Wird der iLP/LLP mit einer Sensoreinheit 150 ausgestattet, kann dieser im Sinne eines Backup-Schrittmachers immer nur dann eine Stimulation abgeben, wenn die gemessene myokardiale Sauerstoffsättigung unter einen kritischen Wert abgesunken ist (absolut oder relativ, gemessen über den Herzzyklus) oder, optional, ein sehr langes RR-Intervall überschritten wird. Als RR-Intervall wird der Abstand zwischen zwei R-Zacken bezeichnet, welche in einem Elektrokardiogramm als Zacken erscheinen und den zeitlichen Abstand zwischen zwei Herzschlägen, d.h. zwischen dem Beginn zweier Kontraktionen der Herzkammern definiert. Ebenso kann in umgekehrter Weise bei einem ratenadaptiven kabellosen Schrittmacher die maximale Stimulationsfrequenz abhängig von der myokardialen Sauerstoffsättigung begrenzt werden, indem bei hohen und ggf. lang anhaltend hohen Stimulationsfrequenzen bei sinkender Sauerstoffsättigung die Stimulationsfrequenz reduziert wird, obwohl ein zweiter, den metabolischen Bedarf signalisierender Sensor zur Ratenadaption (z.B. Aktivitätssensor) weiterhin höhere Stimulationsfrequenzen vorgibt. Hiermit kann eine schrittmacherinduzierte Dekompensation vermieden werden.

Mit der die Sensoreinheit 150 umfassenden Vorrichtung 100 kann eine Therapieoptimierung erfolgen, wobei eine kontinuierliche Messung über einen physiologischen Zyklus hinweg möglich ist, etwa über einen Herzzyklus, einen Atemzyklus und dergleichen. Durch die Kopplung mit einem oder mehreren therapeutischen Implantaten und/oder einem oder mehreren weiteren Sensorimplantaten kann individuell und präzise eine therapeutische Maßnahme und/oder Stimulation erfolgen. Die gemessenen Oxymetriedaten können über ein automatisches Telemetriesystem an einen Arzt und/oder den Patienten und/oder andere Personen und/oder an ein automatisches Auswertesystem gesendet werden.

Durch die Kopplung der Sensoreinheit 150 mit der Steuereinheit (190 in Figur 1) kann eine Optimierung von einem oder mehreren Stimulations- und/oder Therapieparameter derart erfolgen, dass sich eine optimale Sauerstoffsättigung bezogen auf einen definierten Belastungszustand, z.B. Herzfrequenz, des Herzens einstellt.

Dabei kann insbesondere in einer weiteren, nicht dargestellten Ausgestaltung die Sensoreinheit 150 zusätzlich oder alternativ mit einer Einrichtung zur Erfassung eines Kontraktionszustands von Körpergewebe im Bereich oder in der Umgebung der Messstrecke 160 gekoppelt sein. So kann z.B. der Kontraktionszustand des Herzens unmittelbar erfasst und mit dem Messsignal der Sensoreinheit 150 korreliert werden.

Eine Optimierung kann beispielsweise über eine Modulation der AV-Zeit erfolgen (atrioventrikuläre Überleitungszeit), welche die Zeitdauer beschreibt, in der eine Erregung vom Sinusknoten über die Herzvorhöfe auf die Herzkammern übergreift. Zusätzlich oder alternativ kann eine Optimierung über eine Modulation der VV-Zeit erfolgen, welche die zeitliche Verzögerung zwischen rechtsventrikulärer und linksventrikulärer Stimulation beschreibt. Zusätzlich oder alternativ kann eine Optimierung über eine Variation des Stimulationsortes und/oder der Stimulationspolarität erfolgen. Zusätzlich oder alternativ kann eine Optimierung über eine Modulation der Stimulationsfrequenz erfolgen. Vorteilhaft kann die Vorrichtung 100 mit Sensoreinheit 150 mit einem Temperatursensor und/oder einem Heizelement gekoppelt sein (nicht dargestellt). Dies erlaubt einerseits eine Korrektur von Temperatureinflüssen bei der Messung von Signalen, welche repräsentativ für einen Sauerstoffgehalt entlang einer Messstrecke 160 sind. Andererseits kann eine vorgegebene Temperatur eingestellt und ein variabler Temperatureinfluss bei der Messung ausgeschlossen werden. Bei einer stufenweisen Veränderung der Temperatur bei der Messung kann untersucht werden, ob eine anomale Reaktion auf eine Temperaturveränderung vorliegt. Die Blutsauerstoffsättigung steigt typischerweise um 0,5% bei einer Temperaturerhöhung von 2°C.
Weiterhin kann durch eine gezielte lokale Temperaturerhöhung eine Gefäßerweiterung erfolgen, so dass Beiträge zum Messsignal aus verschiedenen Bereichen gezielt hervorgehoben oder unterdrückt werden können.
In einer nicht dargestellten Ausgestaltung kann die Sensoreinheit 150 mit Heizelement, Temperatursensor und Stimulationselektroden gekoppelt sein. Heizelement und Temperatursensor können zwischen Sender 152 und Empfänger 154 positioniert sein, während Sender 152 und Empfänger 154 zwischen eng benachbarten zwei oder mehr Stimulationselektroden (z.B. zwischen Ringelektrode und Fixierhelix oder Tip-Elektrode und Fixierhelix) in einer kompakten Anordnung positioniert sein können. Die Stimulationselektroden sind mit einer entsprechenden Elektronik verbunden, um gezielt Stimulationsimpulse auszusenden.

Bei einem Verfahren zur Bestimmung eines Sauerstoffgehalts in einem Körpergewebe oder im Blut eines menschlichen oder tierischen Körpers, wird eine sauerstoffabhängige Größe bezogen auf einen Sauerstoffgehalt im Gewebe oder im Blut zumindest über einen Herzzyklus erfasst und mit einem Kontraktionszustand des Herzens korreliert. Eine geeignete Vorrichtung mit Sensoreinheit ist in den Figuren 1 bis 3 beschrieben. Jedoch können auch andere Vorrichtungen und/oder Sensoreinheiten eingesetzt werden.

Aus der sauerstoffabhängigen Größe kann eine maximale myokardiale Sauerstoffsättigung, eine minimale myokardiale Sauerstoffsättigung und eine Differenz zwischen minimaler und maximaler myokardialer Sauerstoffsättigung bestimmt und daraus eine Sauerstoffextraktionsrate des Herzens abgeleitet werden.

Die Sauerstoffextraktionsrate wird mittels eines implantierbaren myokardialen Sauerstoffsensors bestimmt, der mit einer Auswerteeinheit verbunden ist, die bezogen auf verschiedene kardiale Funktionszustände (z.B. Frequenz, AV-Zeiten, intrinsische VV-Zeiten bzw. QRS-Signalbreite, VES (ventrikuläre Extrasystolen), VT (ventrikuläre Tachykardie), Afib (atriale Fibrillation)) mindestens die Differenz zwischen maximaler und minimaler Sauerstoffsättigung SpO₂ bestimmt und damit einen Rückschluss auf die myokardiale Extraktionsrate ermöglicht. So kann eine Verlaufskontrolle für Patienten mit chronischer koronarer Herzerkrankung und eingeschränkter Pumpfunktion erfolgen.

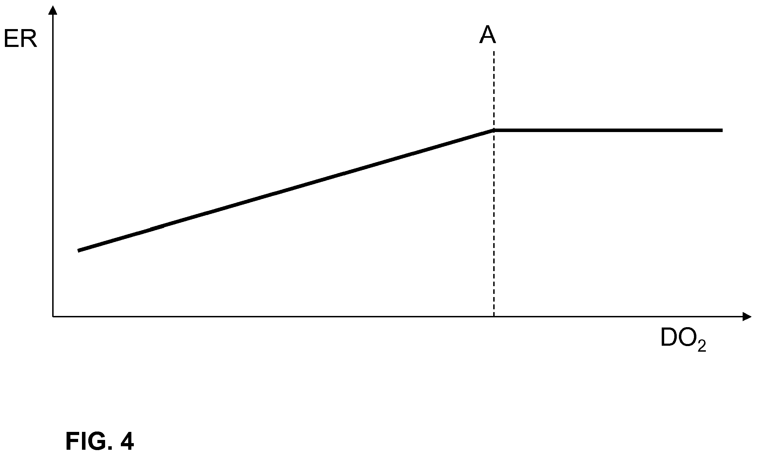

Figur 4 zeigt einen Verlauf einer myokardialen Sauerstoffextraktionsrate ER in Abhängigkeit eines sinkenden Sauerstoffangebots DO₂, d.h. das Sauerstoffangebot DO₂ nimmt in der Figur von links nach rechts ab. Bei sinkendem Sauerstoffangebot DO₂ steigt zunächst die Extraktionsrate an, bis ein gewebetypisches Maximum A erreicht wird. Danach verläuft die Extraktionsrate ER konstant.

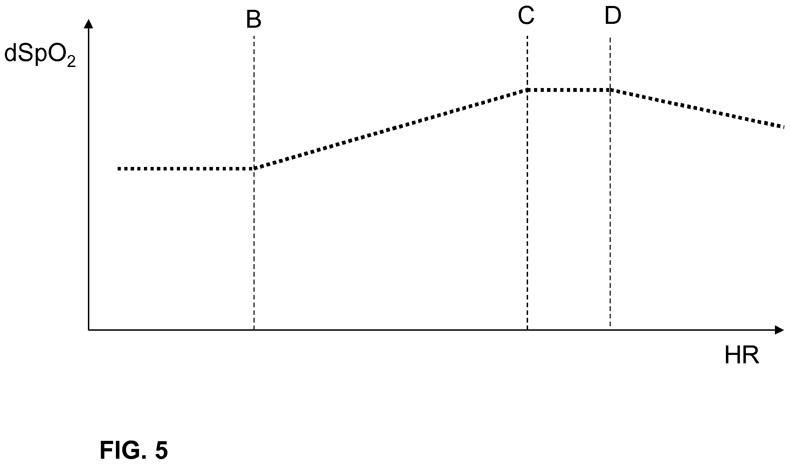

Fig. 5 zeigt einen Verlauf einer Sauerstoffextraktionsratenmessgröße dSpO₂ in Abhängigkeit der Herzfrequenz HR, d.h. die Herzfrequenz HR nimmt in der Figur von links nach rechts zu. Die Messgröße ist dabei die Differenz der minimalen und der maximalen Gewebesauerstoffsättigung SpO₂ im Myokard (gemessen mit einem Gewebesauerstoffsensor, wie z.B. in Figuren 1 bis 3 beschrieben).

Solange bei steigender Herzfrequenz HR auch das Sauerstoffangebot durch gesteigerte Koronardurchblutung ansteigt, bleibt die Messgröße bis zu einer Herzfrequenz B annähernd gleich, d.h. auch die Extraktionsrate dSpO₂ steigt nicht. Beginnt nun durch die Herzinsuffizienz bei weiterer Herzfrequenzsteigerung (B) das Sauerstoffangebot zu stagnieren, wird ein Ansteigen der Extraktionsrate dSpO₂ auch zu einem Anstieg der Messgröße führen. Wenn die maximale Myokard-Sauerstoffextraktionsrate erreicht ist (Punkt C in der Figur), wird mit weiter steigender Herzfrequenz HR ein Plateau in der Messgröße registriert. Dieses Plateau zwischen den Werten C und D der Herzfrequenz HR kann bereits als Signal einer Myokardhypoxie gedeutet werden. Bei weiter ansteigender Herzfrequenz HR oder Phasen lang andauernder hoher Herzfrequenz HR wird durch die zunehmend eingeschränkte Pumpleistung, hervorgerufen durch die Hypoxie, die koronare Perfusion absinken und die Messgröße beginnt ebenfalls zu sinken. Diese Phase signalisiert eine akute Dekompensation

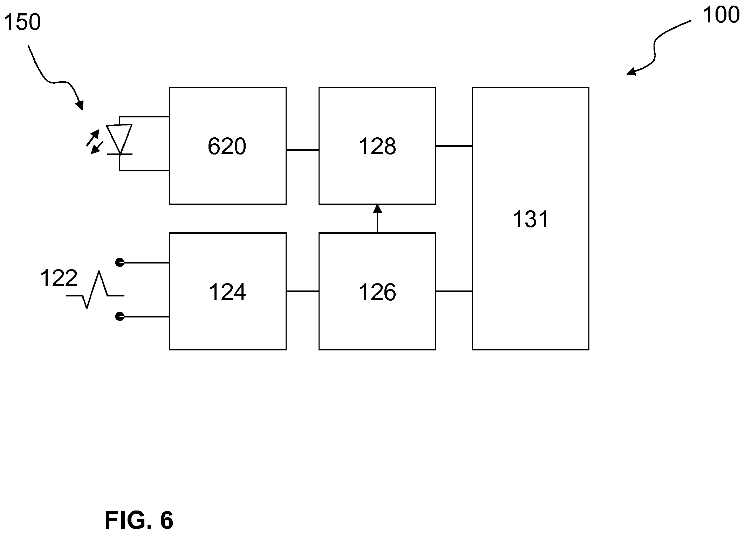

Figur 6 zeigt ein Blockschaltbild einer Vorrichtung 100 mit einer Sensoreinheit 150 nach einem Ausführungsbeispiel der Erfindung, die als Extraktionsratensensor ausgebildet ist. Die Sensoreinheit 150 kann als Oxymetriesensor z.B. entsprechend den Figuren 1 bis 3 ausgebildet sein.

Die myokardiale Sauerstoffsättigung wird durch die dauerhaft implantierte optische Sensoreinheit 150 kontinuierlich in einer mit dem Sensor verbundenen Auswerteeinheit 620 erfasst. Zusätzlich ist das Implantat mit einem EKG/IEGM-Elektrodeninterface 122 ausgestattet, das in einem EKG-Verstärker/Digitalisierer/Filter 124 die EKG-Signale des Herzens ableitet, um aus diesen Signalen in einer Klassifiziereinheit 126 die einzelnen Herzzyklen und deren Phasen (Beginn Systole, Beginn Diastole, etc.) zu identifizieren. Die Phasen des Herzzyklus werden einer Differenzbildungseinheit 128 signalisiert, um so die Differenz der Sauerstoffsättigung innerhalb der einzelnen Herzzyklen zu erfassen. Diese Information und die weiteren von der Klassifizierungseinheit 126 ermittelten Herzzustände werden in der Auswerteeinheit 131 gespeichert und optional in Sinne der Figur 5 ausgewertet. Zusätzlich oder alternativ kann eine Übertragung dieser Daten an ein externes Auswertesystem erfolgen.

Die Auswerteeinheit 620 bestimmt aus einem Verlauf der gemessenen Sauerstoffextraktionsrate über der Herzfrequenz einen kritischen Punkt der Sauerstoffextraktionsrate, bei dem die Sauerstoffextraktionsrate bei steigender Herzfrequenz absinkt und/oder wenn die Sauerstoffextraktionsrate bei steigender Herzfrequenz in einen Sättigungsbereich übergeht und/oder wenn die Sauerstoffextraktionsrate bei steigender Herzfrequenz in einen Sättigungsbereich übergeht und gleichzeitig der maximale Sauerstoffsättigungswert absinkt.

Die Auswerteeinheit 620 ist gleichzeitig mit einer Elektrodenleitung (nicht dargestellt) zur Wahrnehmung der elektrischen Aktivität des Herzens verbunden, um so die kardiale Zykluslänge zu bestimmen.

Die Erfindung ermöglicht es, mit einem implantierbaren Sensor die systolische myokardiale Sauerstoffsättigung kontinuierlich zu überwachen und insbesondere bei herzinsuffizienten Patienten den Therapiebedarf entsprechend anzupassen. Die in Figur 6 beschriebene Vorrichtung ist in der Lage, eine bereits deutlich ausgeprägte Störung der myokardialen Sauerstoffversorgung zu erkennen.

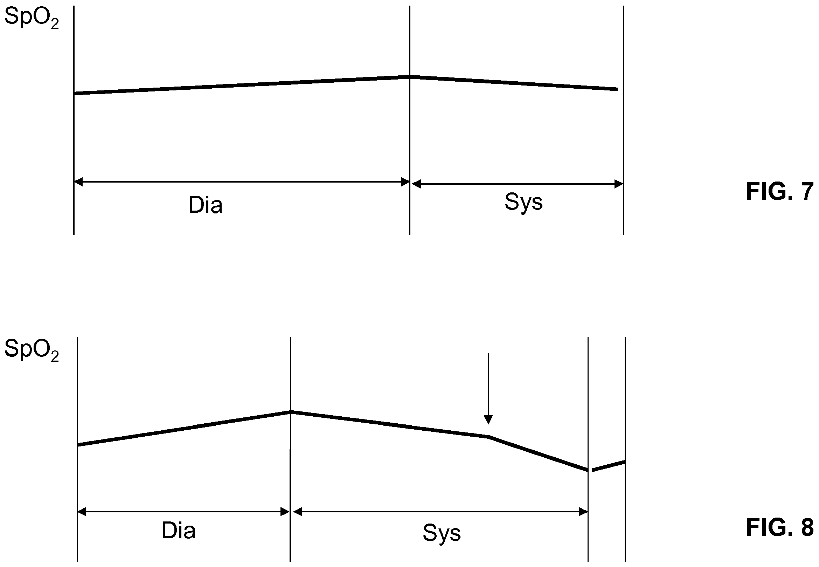

Figur 7 zeigt einen Verlauf einer myokardialen Sauerstoffsättigung SpO₂ in Abhängigkeit eines Kontraktionszustands des Herzens in einem nichthypoxischen Bereich. Figur 8 zeigt einen Verlauf einer myokardialen Sauerstoffsättigung SpO₂ in Abhängigkeit eines Kontraktionszustands desHerzens in einem hypoxischen Bereich.

Während der Diastole Dia in Figur 7 steigt die Sauerstoffsättigung SpO₂ tendenziell an, da hier die Perfusion den Sauerstoffverbrauch im Myokard überkompensiert. Während der Systole Sys wird mehr Sauerstoff verbraucht, als über die nun zusätzlich etwas eingeschränkte Perfusion zugeführt werden kann. Die Einschränkung der Perfusion ist bedingt durch eine Kompression der kleinen Koronararterien während der Systole Sys, zunehmend mit der Dauer und der Intensität der Kontraktion.

In Figur 8 ist ebenfalls ein leichter Anstieg der Sauerstoffsättigung SpO₂ während der Diastole Dia zu sehen. Allerdings sind hier nun bei erhöhter Belastung die Dauer und Intensität der Systole Sys vergrößert, so dass nach einem moderaten Absinken der Sauerstoffsättigung SpO₂ während der Systole Sys im endsystolischen Abschnitt ein überproportionales Absinken des Sauerstoffangebotes auf Grund der verlängerten Kontraktion und weiter eingeschränkten Perfusion zu beobachten ist. Der Übergang ist in der Figur mit einem vertikalen Pfeil gekennzeichnet. Diese drastische Reduktion der Sauerstoffsättigung ist als myokardiale Hypoxie einzustufen und führt bei herzinsuffizienten Patienten zu einer entsprechenden Belastungseinschränkung und im weiteren Verlauf zur Dekompensation und begleitenden kardialen Funktionsstörungen.

Die Erfindung erlaubt es, übermäßigen Sauerstoffmangel zu erkennen und zu signalisieren. Es ist dabei von Vorteil, dass für diese Verlaufskontrolle keine Absolutwertmessung notwendig ist, sondern ein relativer Vergleich innerhalb des kardialen Zyklus ausreichend ist.

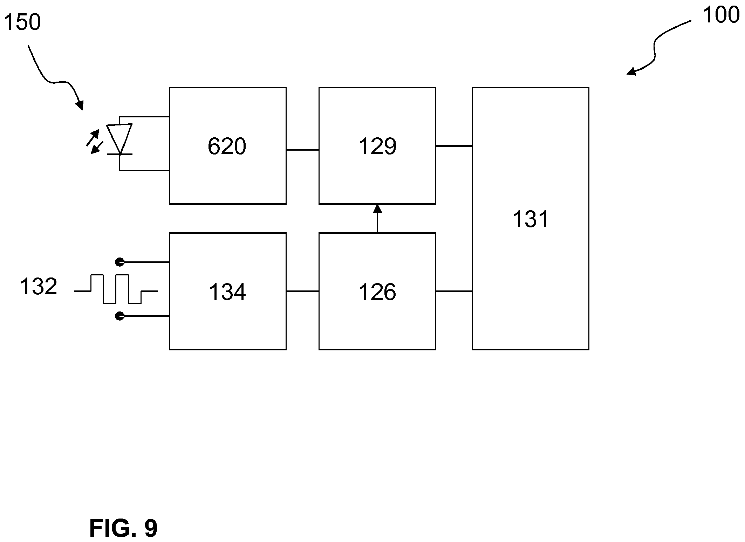

Figur 9 zeigt ein Blockschaltbild einer Vorrichtung 100 mit einer Sensoreinheit 150 nach einem Ausführungsbeispiel der Erfindung zur Messung einer Sauerstoffextraktionsrate. Die Sensoreinheit 150 kann als Oxymetriesensor z.B. entsprechend den Figuren 1 bis 3 ausgebildet sein.

Die myokardiale Sauerstoffsättigung wird durch die dauerhaft implantierte optische Sensoreinheit 150 kontinuierlich in einer mit der Sensoreinheit 150 verbundenen Auswerteeinheit 620 erfasst. Zusätzlich ist das Implantat mit einem mechanischen Kontraktionserfassungssystem 132 ausgestattet, das in diesem Beispiel mittels einer unipolaren, kontinuierlichen Impedanzmessung 134 den mechanischen Kontraktionszustand des Herzens (z.B. nach dem Prinzip der Closed Loop Stimulation CLS: Hier wird die unterschiedliche Leitfähigkeit von Blut und Myokardgewebe genutzt, um so den Kontraktionszustand des Herzens über einen Herzzyklus aufzuzeichnen. Im Augenblick der Diastole befindet sich relativ viel Blutvolumen um die Messelektrode im Ventrikel und die gemessene Impedanz ist auf Grund der besseren Leitfähigkeit des Blutes verhältnismäßig niedrig, bei der Systole nimmt der Einfluss des umgebenden Myokardgewebes zu und die Impedanz steigt an. CLS wird derzeit zur ratenadaption für Schrittmacher und ICDs genutzt) erfasst, um aus diesen Signalen in einer Klassifiziereinheit 126 Beginn und Ende der Kontraktionszustände mechanisch zu bestimmen. Die Phasen der mechanischen Pumpfunktion werden der Sauerstoffsättigung zeitlich in einer Synchronisationseinheit 129 zugeordnet. In der Auswerteeinheit 131 wird anschließend der Sauerstoffverlauf hinsichtlich eines Hypoxiekriterium (z.B. übermäßiges Absinken am Ende der Systole bezogen auf den mittleren Sauerstoffwert über den gesamten Herzzyklus) ausgewertet und signalisiert.

Im Gegensatz zu dem in Figur 6 beschriebenen Ausführungsbeispiel ist die in Figur 9 beschriebene Vorrichtung 100 so konzipiert, dass bereits der Beginn einer myokardialen Sauerstoffminderversorgung angezeigt werden kann und damit eine sehr frühzeitige klinische Interventionsmöglichkeit besteht.

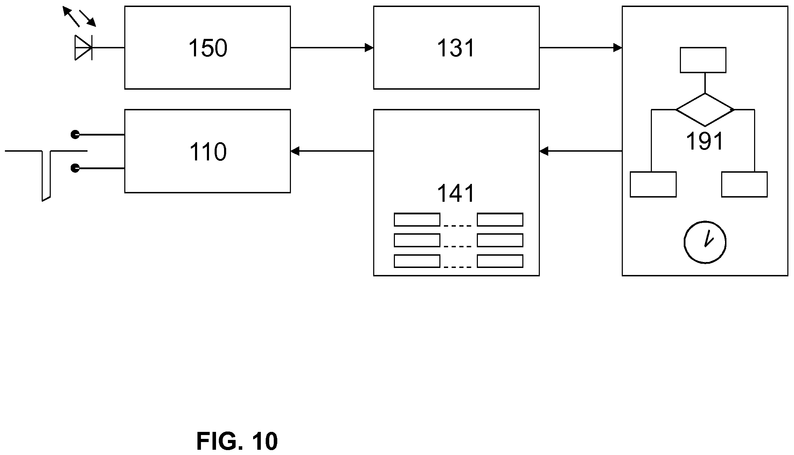

Figur 10 zeigt ein Blockschaltbild eines Herzunterstützungssystems 200 mit einer Sensoreinheit 150, das in Abhängigkeit von der myokardialen Sauerstoffsättigung des Herzens, des Kontraktionszustandes des Herzens und des Herzrhythmus gesteuert wird.

Das Herzunterstützungssystem 200 umfasst eine Sensoreinheit 150 die als Oxymetriesensor z.B. entsprechend den Figuren 1 bis 3 ausgebildet sein kann. Die Sensoreinheit 150 ist mit einem EKG/IEGM-Elektrodeninterface 122 ausgestattet, das in einem EKG/IEGM-Verstärker/Digitalisierer/Filter 124 die EKG/IEGM-Signale des Herzens ableitet, um aus diesen Signalen in einer Klassifiziereinheit 126 die einzelnen Herzzyklen zu identifizieren und die Herzrate zu ermitteln. Die Sensoreinheit 150 ist darüber hinaus mit einem mechanischen Kontraktionserfassungssystem 132 ausgestattet, das in diesem Beispiel mittels einer unipolaren, kontinuierlichen Impedanzmessung den mechanischen Kontraktionszustand des Herzens (z.B. nach dem Prinzip der Closed Loop Stimulation CLS) erfasst, um aus diesen Signalen in einer weiteren Klassifiziereinheit 126 Beginn und Ende der mechanischen Kontraktionszustände zu bestimmen. Alternativ oder zusätzlich kann das mechanische Kontraktionserfassungssystem 132 einen oder mehrere Dehnungsmessstreifen, einen oder mehrere Drucksensoren, einen oder mehrere Beschleunigungssensoren aufweisen. Die erweiterte Sensoreinheit liefert kontinuierlich die Daten zur myokardialen Sauerstoffsättigung, zum Herzrhythmus und zum Kontraktionszustand des Herzens an eine Auswerteeinheit 131, die die drei Datenquellen miteinander korreliert und die gemessenen Sauerstoffsättigungskurven den funktionalen Zuständen des Herzzyklus herzfrequenzabhängig zuordnet.

Weiterhin ist das dargestellte Herzunterstützungssystem 200 mit einer mehrkanaligen kardialen Stimulationseinheit 110 ausgestattet. Diese Stimulationseinheit 110 ist mit einer Zeitsteuerung 141 verbunden, die mehrere, in programmierbaren Grenzen variable Parameter zur Zeitsteuerung bei der Stimulation (z.B. AV-Zeit, VV-Zeit, maximale Herzrate/Stimulationsfrequenz, etc.) vorhält. Ferner umfasst das Herzunterstützungssystem 200 eine Steuereinheit 191, die derart ausgeführt ist, dass diese zyklisch in programmierbaren zeitlichen Intervallen einen Testalgorithmus startet, der jeweils einen oder mehrere Parameter der Zeitsteuerung 141 variiert und dabei die Messkurven der myokardialen Sauerstoffsättigung auswertet. In Abhängigkeit dieser Auswertung werden durch hinterlegte Algorithmen in der Steuereinheit 191 für verschiedene Belastungszustände des Herzens die optimalen Parameter der Zeitsteuerung 141 berechnet und bis zur Durchführung des nächsten Testalgorithmus in der Zeitsteuerung 141 genutzt. Darüber hinaus ist es möglich, zusätzliche Sensorparameter über den metabolischen Bedarf des Patienten oder dessen Aktivitätszustand in die Optimierung der Parameter zur Zeitsteuerung derart einzubeziehen, dass es für verschiedene Belastungszustände angepasste Stimulationsparametersätze gibt.

## Patentansprüche

1. Vorrichtung (100) zum Einführen in den menschlichen oder tierischen Körper, umfassend wenigstens eine dauerimplantierbare Sensoreinheit (150) mit Sender (152) und Empfänger (154) zum Detektieren eines Signals, welches repräsentativ für einen Sauerstoffgehalt entlang einer Messstrecke (160) zwischen Sender (152) und Empfänger (154) ist, wobei vorrichtungsseitig ein Reflektor (156) vorgesehen ist, der zur Reflexion des Signals in der Messstrecke (160) zwischen Sender (152) und Empfänger (154) angeordnet ist, **gekennzeichnet durch** Anordnung des Reflektors (156) an oder in einer Fixiervorrichtung (230) zur dauerhaften Fixierung der die Sensoreinheit (150) umfassenden Vorrichtung (100) an oder in einem Körpergewebe.

2. Vorrichtung nach Anspruch 1, wobei die Sensoreinheit (150) zusätzlich mit einer Elektrodenleitung zur Wahrnehmung der elektrischen Aktivität des Herzens verbunden ist, um so die kardiale Zykluslänge bestimmen zu können wodurch das Signal, welches repräsentativ für einen Sauerstoffgehalt ist, jeweils für einen Herzzyklus und/oder bestimmte Phasen eines Herzzyklus ermittelt werden kann.

3. Vorrichtung nach einem der vorhergehenden Ansprüche, wobei die Sensoreinheit (150) mit einem oder mehreren therapeutischen Implantaten gekoppelt oder koppelbar ist.

4. Vorrichtung nach einem der vorhergehenden Ansprüche, umfassend die Sensoreinheit (150) sowie eine mit der Sensoreinheit (150) gekoppelte Therapieeinheit (110, 120, 130, 140).

5. Vorrichtung nach einem der vorhergehenden Ansprüche, wobei die Sensoreinheit (150) Bestandteil einer Therapieeinheit (110, 120, 130, 140) ist, welche wenigstens eine Wahrnehmungselektrode und/oder eine therapeutische Stimulationselektrode und/oder eine Defibrillationselektrode umfasst.

6. Vorrichtung nach einem der vorhergehenden Ansprüche, wobei die Sensoreinheit (150) mit einer Einrichtung zur Erfassung eines pH-Werts gekoppelt ist.

7. Vorrichtung nach einem der vorhergehenden Ansprüche, wobei die Sensoreinheit (150) mit einem Temperatursensor und einem Heizelement gekoppelt ist.

8. Vorrichtung nach einem der vorhergehenden Ansprüche, wobei die Sensoreinheit (150) mit einer Einrichtung zur Erfassung eines Kontraktionszustands von Körpergewebe im Bereich der Messstrecke (160) gekoppelt ist.

9. Vorrichtung nach einem der vorhergehenden Ansprüche, wobei die Vorrichtung derart eingerichtet ist, dass aus dem Signal, welches repräsentativ für einen Sauerstoffgehalt ist, eine maximale myokardiale Sauerstoffsättigung, eine minimale myokardiale Sauerstoffsättigung und eine Differenz zwischen minimaler und maximaler myokardialer Sauerstoffsättigung bestimmt werden können und daraus eine Sauerstoffextraktionsrate des Herzens abgeleitet werden kann.

10. Vorrichtung nach einem der vorhergehenden Ansprüche, wobei die Vorrichtung derart eingerichtet ist, dass aus dem Signal, welches repräsentativ für einen Sauerstoffgehalt ist, die maximale, minimale und die Differenz der myokardialen Sauerstoffsättigung über einen Herzzyklus bestimmt werden können und daraus ein Verlauf einer Sauerstoffextraktionsrate in Abhängigkeit der Herzfrequenz bestimmt werden kann.

11. Vorrichtung nach Anspruch 10, wobei die Vorrichtung derart eingerichtet ist, dass aus einem Verlauf der gemessenen Sauerstoffextraktionsrate über der Herzfrequenz ein kritischer Punkt der Sauerstoffextraktionsrate bestimmt werden kann, bei dem die Sauerstoffextraktionsrate bei steigender Herzfrequenz absinkt; und/oder wenn die Sauerstoffextraktionsrate bei steigender Herzfrequenz in einen Sättigungsbereich übergeht; und/oder wenn die Sauerstoffextraktionsrate bei steigender Herzfrequenz in einen Sättigungsbereich übergeht und gleichzeitig der maximale Sauerstoffsättigungswert absinkt.

12. Vorrichtung nach Anspruch 10, wobei die Vorrichtung derart eingerichtet ist, dass ein kritischer Punkt der Sauerstoffextraktionsrate bestimmt werden kann, ab dem eine systolische Sauerstoffsättigung unter einen kritischen Referenzwert absinkt oder die Absinkgeschwindigkeit einen kritischen Wert während der Systole übersteigt.

13. Herzunterstützungssystem (200) umfassend eine Vorrichtung (100) nach einem der vorhergehenden Ansprüche, wenigstens eine Therapieeinheit (110) sowie eine Steuereinheit (190), wobei die Steuereinheit (190) zur Optimierung von einem oder mehreren Stimulations- und/oder Therapieparametern so ausgelegt ist, dass eine Sauerstoffsättigung im Bereich der Messstrecke (160) bezogen auf einen definierten Belastungszustand des Herzens einstellbar ist.

14. Herzunterstützungssystem nach Anspruch 13, wobei die Sensoreinheit (150) mit einer Einrichtung zur Erfassung eines Kontraktionszustands des Herzens gekoppelt ist.

15. Herzunterstützungssystem nach Anspruch 13 oder 14, wobei ein oder mehrere Stimulations- und/oder Therapieparameter derart optimiert werden können, dass eine Sauerstoffsättigung bezogen auf einen definierten Belastungszustand des Herzens eingestellt wird.

## Claims

1. A device (100) for insertion into the human or animal body, comprising at least one permanently implantable sensor unit (150) with transmitter (152) and receiver (154) for detecting a signal representative for an oxygen content along a measurement path (160) between the transmitter (152) and receiver (154), wherein a reflector (156) is provided on the device, said reflector being arranged in the measurement path (160) between the transmitter (152) and receiver (154) for reflection of the signal,
**characterised by** arrangement of the reflector (156) on or in a fixing device (230) for permanently fixing the device (100) comprising the sensor unit (150) to or in a bodily tissue.

2. The device according to claim 1, wherein the sensor unit (150) is connected additionally to an electrode line for sensing the electrical activity of the heart so as to be able to determine the cardiac cycle length, whereby the signal representative for an oxygen content can be determined for a cardiac cycle and/or certain phases of a cardiac cycle.

3. The device according to either one of the preceding claims, wherein the sensor unit (150) is coupled or can be coupled to one or more therapeutic implants.

4. The device according to any one of the preceding claims, comprising the sensor unit (150) and also a therapy unit (110, 120, 130, 140) coupled to the sensor unit (150).

5. The device according to any one of the preceding claims, wherein the sensor unit (150) is part of a therapy unit (110, 120, 130, 140), which comprises at least one sensing electrode and/or a therapeutic stimulation electrode and/or a defibrillation electrode.

6. The device according to any one of the preceding claims, wherein the sensor unit (150) is coupled to an arrangement for detecting a pH value.

7. The device according to any one of the preceding claims, wherein the sensor unit (150) is coupled to a temperature sensor and a heating element.

8. The device according to any one of the preceding claims, wherein the sensor unit (150) is coupled to an arrangement for detecting a contraction state of bodily tissue in the region of the measurement path (160).

9. The device according to any one of the preceding claims, wherein the device is designed in such a way that a maximum myocardial oxygen saturation, a minimum myocardial oxygen saturation, and a difference between minimum and maximum myocardial oxygen saturation can be determined from the signal representative for an oxygen content, and from this an oxygen extraction rate of the heart can be derived.

10. The device according to any one of the preceding claims, wherein the device is designed in such a way that the maximum, minimum, and the difference of the myocardial oxygen saturation over a cardiac cycle can be determined from the signal representative for an oxygen content, and from this a course of an oxygen extraction rate can be determined in accordance with the heart rate.

11. The device according to claim 10, wherein the device is designed in such a way that a critical point of the oxygen extraction rate can be determined from a course of the measured oxygen extraction rate over the heart rate, at which point the oxygen extraction rate decreases with rising heart rate; and/or when the oxygen extraction rate with rising heart rate transitions into a saturation range; and/or when the oxygen extraction rate with rising heart rate transitions into a saturation range and at the same time the maximum oxygen saturation value decreases.

12. The device according to claim 10, wherein the device is designed in such a way that a critical point of the oxygen extraction rate can be determined, from which a systolic oxygen saturation falls below a critical reference value or the rate of decrease exceeds a critical value during the systole.

13. A cardiac assist system (200) comprising a device (100) according to any one of the preceding claims, comprising at least one therapy unit (110), and a control unit (190), wherein the control unit (190) is configured to optimise one or more stimulation and/or therapy parameters, such that an oxygen saturation in the region of the measurement path (160) with regard to a defined state of load of the heart can be set.

14. The cardiac assist system according to claim 13, wherein the sensor unit (150) is coupled to an arrangement for detecting a contraction state of the heart.

15. The cardiac assist system according to claim 13 or 14, wherein one or more stimulation and/or therapy parameters can be optimised in such a way that an oxygen saturation with regard to a defined state of load of the heart is set.

## Revendications

1. Dispositif (100) destiné à l'insertion dans le corps humain ou animal, comprenant au moins une unité de détection (150) implantable à demeure avec un émetteur (152) et un récepteur (154) pour la détection d'un signal, lequel est représentatif pour une teneur en oxygène le long d'un trajet de mesure (160) entre l'émetteur (152) et le récepteur (154), où un réflecteur (156) est prévu du côté du dispositif qui est placé pour la réflexion du signal sur le trajet de mesure (160) entre l'émetteur (152) et le récepteur (154),
**caractérisé par** un agencement du réflecteur (156) sur ou dans un dispositif de fixation (230) pour la fixation à demeure du dispositif (100) comprenant l'unité de détection (150) sur ou dans un tissu corporel.

2. Dispositif selon la revendication 1, dans lequel l'unité de détection (150) est reliée en outre avec une ligne d'électrode pour la perception de l'activité électrique du coeur, afin de pouvoir ainsi déterminer la durée de cycle cardiaque, ce par quoi le signal, lequel est représentatif d'une teneur en oxygène, peut déterminer respectivement, un cycle cardiaque et/ou des phases définies d'un cycle cardiaque.

3. Dispositif selon l'une des revendications précédentes, dans lequel l'unité de détection (150) est couplée ou peut être couplée avec un ou plusieurs implants thérapeutiques.

4. Dispositif selon l'une des revendications précédentes, comprenant l'unité de détection (150), ainsi que l'unité de thérapie (110, 120, 130, 140) couplée avec l'unité de détection (150).

5. Dispositif selon l'une des revendications précédentes, ans lequel l'unité de détection (150) est un composant d'une unité de thérapie (110, 120, 130, 140), laquelle comprend au moins une électrode de détection et/ou une électrode de stimulation thérapeutique et/ou une électrode de défibrillation.

6. Dispositif selon l'une des revendications précédentes, dans lequel l'unité de détection (150) est couplée avec un dispositif pour l'évaluation d'une valeur de pH.

7. Dispositif selon l'une des revendications précédentes, dans lequel l'unité de détection (150) est couplée avec un capteur de température et un élément chauffant.

8. Dispositif selon l'une des revendications précédentes, dans lequel l'unité de détection (150) est couplée avec un dispositif pour la détection d'un état de contraction de tissus corporels dans la région du trajet de mesure (160).

9. Dispositif selon l'une des revendications précédentes, où le dispositif est conçu de telle manière qu'à partir du signal, lequel est représentatif d'une teneur d'oxygène, une saturation en oxygène myocardiale maximale, une saturation en oxygène myocardiale minimale et une différence entre la saturation en oxygène minimale et maximale peuvent être déterminées, et un taux d'extraction d'oxygène du coeur peut en être déduit.

10. Dispositif selon l'une des revendications précédentes, où le dispositif est conçu de telle manière qu'à partir du signal, lequel est représentatif d'une teneur d'oxygène, la saturation en oxygène myocardiale maximale, minimale et la différence de saturation en oxygène minimale et maximale sur un cycle cardiaque peuvent être déterminées, et une évolution d'un taux d'extraction d'oxygène peut être déduite en fonction de la fréquence cardiaque.

11. Dispositif selon la revendication 10, où le dispositif est conçu de telle manière, qu'à partir d'une évolution des taux d'extraction de l'oxygène en fonction de la fréquence cardiaque, un point critique du taux d'extraction de l'oxygène peut être déterminé pour lequel le taux d'extraction de l'oxygène diminue lorsque la fréquence cardiaque est croissante ; et/ou si le taux d'extraction de l'oxygène dépasse un domaine de saturation pour une fréquence cardiaque croissante ; et/ou si le taux d'extraction de l'oxygène dépasse un domaine de saturation pour une fréquence cardiaque croissante et que la valeur de saturation en oxygène maximale diminue simultanément.

12. Dispositif selon la revendication 10, où le dispositif est conçu de telle manière qu'un point critique du taux d'extraction de l'oxygène peut être déterminé, à partir duquel une saturation en oxygène systolique diminue en dessous d'une valeur de référence critique ou que la vitesse de diminution dépasse une valeur critique pendant la systole.

13. Système d'assistance cardiaque (200) comprenant un dispositif (100) selon l'une des revendications précédentes, au moins une unité de thérapie (110) ainsi qu'une unité de commande (190), où l'unité de commande (190) est prévue pour l'optimisation d'un ou de plusieurs paramètres de stimulation et/ou de thérapie de sorte qu'une saturation d'oxygène dans la région d'un trajet de mesure (160) peut être réglée par rapport à un état de sollicitation défini du coeur.

14. Système d'assistance cardiaque selon la revendication 13, dans lequel l'unité de détection (150) est couplée avec un dispositif pour la détection d'un état de contraction du coeur.

15. Système d'assistance cardiaque selon la revendication 13 ou 14, dans lequel un ou plusieurs paramètres de stimulation et/ou de thérapie peuvent être optimisés de telle manière qu'une saturation en oxygène est réglée pour un état de sollicitation défini du coeur.
